# EUROPÄISCHE PATENTSCHRIFT

(11) **EP 3 205 361 B1**
(45) Veröffentlichungstag und Bekanntmachung des Hinweises auf die Patenterteilung: **26.05.2021**
(21) Anmeldenummer: 17153376.3
(22) Anmeldetag: 26.01.2017
(51) Int. Cl.: A61M 1/14

(54) **MASCHINE ZUR EXTRAKORPORALEN BLUTBEHANDLUNG MIT LICHTGEBENDER EINRICHTUNG**
MACHINE FOR EXTRACORPOREAL BLOOD TREATMENT WITH LIGHTING DEVICE
MACHINE DESTINÉE AU TRAITEMENT SANGUIN EXTRACORPOREL AVEC UN DISPOSITIF LUMINEUX

(30) Priorität: 11.02.2016 DE 102016102353
(43) Veröffentlichungstag der Anmeldung: 16.08.2017
(73) Patentinhaber: B. Braun Avitum AG, 34212 Melsungen (DE)
(72) Erfinder: SCHÄFER, Oliver, 36286 Neuenstein (DE); SCHLEICHER, Christian, 36160 Dipperz (DE); MÖLLER, Dirk, 34326 Altmorschen (DE)
(74) Vertreter: Winter, Brandl - Partnerschaft mbB

(56) Entgegenhaltungen:
- EP-A1- 2 371 411
- DE-A1-102013 005 743
- DE-U1- 29 805 006
- US-A1- 2003 086 817
- US-A1- 2012 170 304

## Beschreibung

Die Erfindung betrifft eine Maschine zur extrakorporalen Blutbehandlung mit einer lichtgebenden Einrichtung, und bezieht sich insbesondere auf eine Maschine zur extrakorporalen Blutbehandlung, die eine Leuchteinrichtung als die lichtgebende Einrichtung zur Anzeige eines Betriebszustands, zur Erzeugung eines Umlichts, einer Raumbeleuchtung und/oder dergleichen beinhaltet.

Maschinen zur extrakorporalen Blutbehandlung wie beispielsweise Dialysemaschinen verfügen häufig über Leuchtmittel, die mehrfarbig oder zur Erzeugung mehrerer Farben ausgebildet sein können, wie beispielsweise Leuchtdioden bzw. LEDs. Solche Leuchtmittel sind üblicherweise dazu angeordnet, abhängig vom Status oder Betriebszustand der Maschine (rot, gelb, grün) auf eine transparente Streuscheibe abzustrahlen, um ein Leuchtfeld zu erzeugen, das von außen sichtbar einen jeweiligen Betriebszustand kenntlich macht. Weiterhin ist das Prinzip einer auf Leuchtdioden basierenden und mit einer Streuscheibe arbeitenden Rundumleuchte bekannt.

Nachteilig ist hierbei unter anderem, dass die Leuchtmittel unmittelbar hinter der Streuscheibe angeordnet sein müssen, um ihr Licht nach außerhalb der Maschine zur extrakorporalen Blutbehandlung abstrahlen zu können, und dass dazu entsprechender Bauraum an genau dieser Stelle benötigt wird und beispielsweise elektrische Leitungen dorthin zu verlegen sind. Die Leuchtmittel leuchten dabei lediglich punktuell an ihrem Einbauort aus. Für eine größere auszuleuchtende Fläche, oder Leuchtgrafiken, ist eine entsprechend große Anzahl von Leuchtmitteln mit entsprechend viel Bauraum und aufwändiger Verkabelung und Ansteuerung vonnöten. Dennoch ist auf diese Weise eine gewünschte Leuchtdichte bzw. Lichtintensität und/oder eine ausreichend gleichmäßige Lichtabgabe oder Lichtverteilung nicht zufriedenstellend erzielbar, und sind bei einem Ausfall einzelner Leuchtmittel Reparaturen aufwändig.

Aus dem Stand der Technik bekannt ist die Druckschrift EP 2 371 411 A1, die ein medizinisches Gerät (z.B. eine Dialysemaschine) mit einem angeschlossenen, leuchtenden Schlauch zeigt, der durch Farbe, Intensität und Position des Lichtsignals einem Anwender einen Therapiezustand anzeigt.

US 2003/0086817 A1 zeigt ein UV-Desinfektionssystem für Blut mit einer UV-Lampe, einer Übertragungsleitung für das UV-Licht und einer Desinfektionskammer.

DE 10 2013 005 743 A1 offenbart ein medizintechnisches Gerät, an dessen Maschinenoberfläche ein transparentes Display angeordnet ist.

US 2012/0170304 A1 beschreibt einen ringförmigen Lichtleiter.

In der Praxis wird außerdem während einer Dialysetherapie eine Dialysemaschine vorzugsweise in der Nähe eines oder neben einem Patienten aufgestellt. Die Dialysemaschine verfügt dabei regelmäßig über ein Bedienfeld zur Steuerung der Maschine, über welches auch verschiedenartige Informationen abrufbar sein können. Ein solches Bedienfeld bzw. dessen Informationsdarstellung ist jedoch in der Regel komplex aufgebaut und/oder detailliert und im Übrigen nicht notwendiger Weise beispielsweise einem Patienten zugewandt.

Eine separate und rasch erfassbare Anzeige, Meldung oder Rückmeldung eines Betriebs- und/oder Therapiezustands kann daher die diesbezüglich relevante Wahrnehmung des Anwenders und/oder des Patienten verbessern und beschleunigen, zumal nicht von jeder mit der Therapie befassten Person oder einem Patienten erwartet werden kann, dass diese(r) in der Lage ist, komplexe Informationen am Bedienfeld korrekt oder rasch zu erfassen.

Der Erfindung liegt daher als eine Aufgabe zugrunde, eine Maschine zur extrakorporalen Blutbehandlung mit einer lichtgebenden Einrichtung zu schaffen, welche lichttechnische Freiheitsgrade zur Darstellung von Zuständen der Dialysemaschine und/oder eines Therapieverlaufs außerhalb eines Bedienfelds und/oder einer Informationsdarstellung auf demselben erhöht.

Diese Aufgabe wird erfindungsgemäß gelöst durch eine Maschine zur extrakorporalen Blutbehandlung mit den Merkmalen des Anspruchs 1. Vorteilhafte Weiterbildungen der Erfindung sind Gegenstand der beigefügten Unteransprüche.

Der Erfindung liegt als eine allgemeine Idee zugrunde, Licht von einer Lichtquelle wie beispielsweise einer Leuchtiode bzw. LED oder OLED an eine beliebige bzw. gewünschte Stelle der Maschine zur extrakorporalen Blutbehandlung zu führen und an dieser Stelle auszugeben, d.h. abzustrahlen.

In einer Ausführungsform kann dazu ein Lichtleiter vorgesehen sein, der aus einem transparenten Material bestehen kann, In diesem Fall kann der Lichtleiter an vorbestimmter Stelle teilweise leuchten, oder kann der gesamte Lichtleiter leuchten, und dadurch eine zumindest teilweise an seinem Umfang bis hin zu rundum abstrahlende lichtgebende Einrichtung (Leuchteinrichtung) bilden. Bei rundum abstrahlender Charakteristik kann der Lichtleiter beispielsweise als eine aus allen Richtungen sichtbare und erfassbare 360°-Statusanzeige und/oder eine als nach dem Prinzip eines Ambient Light Umlicht erzeugende Leuchteinrichtung, die etwa einen Therapiezustand oder einen Betriebszustand der Dialysemaschine auf eine beliebige Oberfläche in der Umgebung der Dialysemaschine projiziert, genutzt werden und dadurch die Wahrnehmung des Anwenders verbessern.

In einer anderen Ausführungsform kann die lichtgebende Einrichtung bzw. der Lichtleiter durch eine selbstleuchtende OLED (Organic Light Emitting Diode)-Anordnung gebildet und dazu angeordnet sein, Informationen, wie beispielsweise einen Betriebszustand der Maschine oder einen Therapiezustand, durch Platzierung an einer beliebigen oder vorbestimmten sichtbaren Geräteoberfläche anzuzeigen. Aufgrund der Eigenschaften der OLED-Technik kann zum einen eine OLED-Einrichtung insgesamt, d.h. die gesamte OLED, leuchten und somit als 360°-Statusanzeige genutzt werden, und/oder zum anderen dazu genutzt werden, Licht an eine beliebige Stelle der Maschine zu führen. Je nach geometrischer bzw. flächiger Ausgestaltung der verwendeten lichtgebenden Einrichtung (Lichtleiter und/oder OLED-Einrichtung) kann dann beispielsweise ein Teil der oder auch die gesamte Maschinenoberfläche aufleuchten, so dass beispielsweise ein Lichtleiter/eine OLED-Einrichtung entsprechend einer umlaufenden Gehäusekontur, eine partielle Ausleuchtung mit seitlich umlaufendem Lichtleiter/seitlich verlaufender OLED-Einrichtung, und/oder ein flächiger Lichtleiter/eine flächige OLED-Einrichtung entsprechend einer Monitor- bzw. Gehäuseoberfläche darstellbar sind.

Die Erfindung ist insbesondere dahingehend vorteilhaft, dass sich eine Lichtquelle nicht unmittelbar hinter einer Streuscheibe befinden muss, sondern je nach verfügbarem Bauraum an einer beliebigen Stelle im Gerät oder als selbstleuchtende Fläche an vorbestimmter Stelle am Gerät angeordnet sein kann. Auf diese Weise können auch Stellen an der Dialysemaschine lichtgebend ausgestaltet werden, die bisher beispielsweise mangels dort zur Verfügung stehendem Bauraum nicht erreichbar sind. Die Erfindung erhöht damit entwicklungsbezogen Freiheitsgrade und erzielt mit geringem Aufwand eine verbesserte Ablesbarkeit und Erkennbarkeit eines Maschinenstatus für einen Anwender.

Im Einzelnen werden die vorstehenden Vorteile realisiert und die Aufgabe gelöst durch eine Maschine zur extrakorporalen Blutbehandlung, mit zumindest einem einen Bestandteil dieser Maschine zur extrakorporalen Blutbehandlung bildenden Gehäuse zur Aufnahme betrieblicher Komponenten der Maschine zur extrakorporalen Blutbehandlung; und einer streuscheibenlos lichtgebenden Einrichtung zur Darstellung eines Betriebs- und/oder Therapiezustands der Maschine zur extrakorporalen Blutbehandlung nach dem Umlichtprinzip, wobei die lichtgebende Einrichtung in vorbestimmtem Verlauf und/oder mit vorbestimmter Formgebung an zumindest einem Abschnitt des Gehäuses angeordnet und ein flexibler Lichtleiter (LLa bis LLe), der zumindest eine stirnseitige Lichteinkoppelfläche an einem ersten Ende und zumindest eine stirnseitige Lichtauskoppelfläche an einem zweiten Ende aufweist, oder eine selbstleuchtende, flächige und/oder flexible OLED-Einrichtung ist. Dabei leuchtet zumindest ein Teil der Maschinenoberfläche auf und die lichtgebende Einrichtung ist dazu angeordnet, mittels Umlicht (Ambient Light) einen Therapiezustand und/oder einen Betriebszustand der Maschine zur extrakorporalen Blutbehandlung an eine Oberfläche in der Umgebung dieser Maschine derart zu projizieren, dass ein Therapiezustand und/oder Betriebszustand an einer durch die lichtgebende Einrichtung erhellten Fläche im Raum erkennbar ist.

Eine OLED-Einrichtung erzeugt vorteilhaft selbst und an Ort und Stelle ausreichend Licht, so dass vorteilhaft eine separat entfernte Lichteinkopplung entfallen kann.

Vorteilhaft kann dadurch ein Lichterzeugungselement, wie beispielsweise eine LED, entfernt von einem gewünschten Lichtausgabeort in dort gut nutzbarem Bauraum angeordnet werden, und kann deren Licht über den Lichtleiter an den gewünschten Lichtausgabeort geführt werden.

Bevorzugt weist der flexible Lichtleiter zumindest einen in radialer Richtung Licht auskoppelnden Abschnitt entlang seines Längsverlaufs auf. Vorteilhaft kann dazu der Lichtleiter so konfiguriert (bearbeitet) sein, dass er Licht an zumindest einer vorbestimmten Stelle und/oder in einer vorbestimmten Richtung, bis hin zu einem radial rundum erfolgenden Leuchten des Lichtleiters, auskoppelt.

Vorteilhaft ist die mittels der lichtgebenden Einrichtung erzielbare Lichtintensität derart, dass zumindest die Umgebung der Maschine zur extrakorporalen Blutbehandlung erhellbar ist.

Bevorzugt ist die lichtgebende Einrichtung dazu angeordnet, in einem gesamten Lichtabgabebereich zu leuchten und eine 360°-Statusanzeige zu bilden. Vorteilhaft können sowohl ein flexibler, rundum leuchtender Lichtleiter als auch eine flexible, d.h. verformbare OLED-Einrichtung zu einem Rundstrahler geformt werden, der Licht in alle Raumrichtungen abstrahlt.

Bevorzugt ist die lichtgebende Einrichtung mit zumindest einem Teil ihrer Ausdehnung entlang einer umlaufenden Gehäusekontur des zumindest einen Gehäuses, an zumindest einer Teilfläche des zumindest eines Gehäuses und/oder gegenüber dem zumindest einen Gehäuse vorstehend angeordnet. Vorteilhaft kann dadurch die Maschine zur extrakorporalen Blutbehandlung mit zumindest einer vorbestimmten Leuchtgrafik versehen werden.

Bevorzugt ist die lichtgebende Einrichtung mit zumindest einem Teil ihrer Ausdehnung innenseitig der Maschine zur extrakorporalen Blutbehandlung entlang eines transparenten oder opaken Abschnitts einer Gehäusewandung des zumindest einen Gehäuses geführt und dazu angeordnet, ausgekoppeltes Licht durch den transparenten oder opaken Abschnitt nach außerhalb der Maschine zur extrakorporalen Blutbehandlung abzustrahlen. Hierdurch kann beispielsweise eine blendfreie Objekt- oder Arbeitsbeleuchtung, wie beispielsweise eine Beleuchtung von Anschlüssen oder Bedienelementen an der Maschine zur extrakorporalen Blutbehandlung, konfiguriert werden.

Bevorzugt bedeckt die lichtgebende Einrichtung zumindest einen Teil einer Gehäuseoberfläche des zumindest einen Gehäuses oder ist in zumindest einen Teil der

Gehäuseoberfläche eingelassen. Das zumindest eine Gehäuse kann dabei ein Gehäuse eines mit einer Rumpfkonfiguration der Maschine zur extrakorporalen Blutbehandlung verbundenen Monitors sein.

Bevorzugt weist die lichtgebende Einrichtung eine vorbestimmte Verlaufs-, Linien- und/oder Flächengrafik auf.

Bevorzugt ist die lichtgebende Einrichtung dazu angeordnet, zumindest abschnittsweise mehrfarbig oder vollflächig einfarbig zu leuchten.

Die Erfindung wird nachstehend anhand bevorzugter Ausführungsbeispiele unter Bezugnahme auf die beigefügte Zeichnung näher beschrieben. Es zeigen:
Fig. 1 in vereinfachter, perspektivischer Darstellung ein Gerätegehäuse einer Dialysemaschine als einem medizinischen Gerät (Maschine) zur extrakorporalen Blutbehandlung;
Fig. 2 das Gerätegehäuse gemäß Fig. 1 mit einem Lichtleiter als lichtgebender Einrichtung gemäß einem ersten bevorzugten Ausführungsbeispiel der Dialysemaschine mit lichtgebender Einrichtung; und
Fig. 3 das Gerätegehäuse gemäß Fig. 1 mit einer OLED-Einrichtung als lichtgebender Einrichtung gemäß einem zweiten bevorzugten Ausführungsbeispiel der Dialysemaschine mit lichtgebender Einrichtung.

In der nachfolgenden Figurenbeschreibung werden in den einzelnen Figuren gleiche oder gleichwirkende Elemente und/oder Komponenten gleich und/oder mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

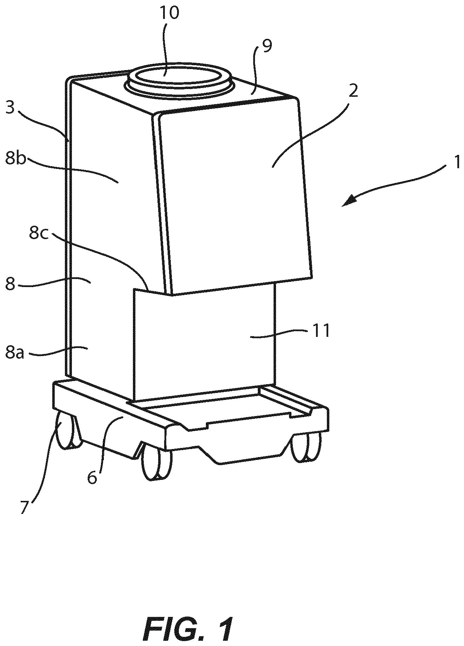

Fig. 1 zeigt in vereinfachter, perspektivischer Darstellung ein Gerätegehäuse einer Dialysemaschine als einem medizinischen Gerät zur extrakorporalen Blutbehandlung. Gemäß Fig. 1 hat die Dialysemaschine ein im Wesentlichen selbsttragendes Geräte- bzw. Maschinengehäuse 1 (nachstehend als Gehäuse bezeichnet), in welchem eine Anzahl von Maschinenteilen oder Maschinenkomponenten zur Durchführung einer Blutbehandlung in an sich bekannter Weise funktionsfähig zusammenwirkend angeordnet sind oder angeordnet werden können, wie beispielsweise eine Steuerelektronik, zumindest Teile der Hydraulik, Pumpen, Heizeinrichtungen und ggf. Tanks oder Beutel für ausgewählte Betriebsstoffe, welche sämtlich aus dem Stand der Technik hinlänglich bekannt sind und daher nicht weiter dargestellt oder beschrieben werden.

Das Gehäuse 1 gemäß Fig. 1 kann beispielsweise im Wesentlichen zumindest zwei Seitenplatten 8 , eine zumindest teilweise als schwenkbare Tür/Klappe ausgebildete Rückwand 3 , eine Haube 9 , welche die beiden (parallel beabstandeten) Seitenplatten 8 oberseitig verbindet, eine Bodenplatte (beispielsweise als Teil eines Gerätesockels) 6, welche die beiden Seitenplatten 8 bodenseitig verbindet, und eine zumindest teilweise als schwenkbare Tür/Klappe 2 ausgebildete Vorderwand aufweisen. Ferner können eine Anzahl von Gelenk- oder Scharniervorrichtungen (nicht dargestellt) an entsprechenden Stellen vorgesehen sein, um ein Öffnen und Schließen der schwenkbaren Tür/Klappe 2 und/oder der Rückwand 3 zu ermöglichen.

An die Haube 9 kann beispielsweise zentral oder mittig ein Anschlusssockel 10 angeformt sein, an welchem ein zusätzliches Gerät wie beispielsweise ein Monitor und/oder ein Bedienfeld (nicht dargestellt) montierbar ist. Die Bodenplatte 6 kann an in Gehäuseumfangsrichtung beabstandeten Positionen Anlenkstellen aufweisen, an denen Geräterollen 7 montierbar/montiert sind, welche zusammen mit der Bodenplatte 6 ein geräteinternes (integriertes) Fahrwerk einer mobilen Dialysemaschine bilden können. Alternativ kann die Bodenplatte 6 auf einem separaten Fahrwerk, beispielsweise einem Rollenwagen, platziert werden, falls keine Geräterollen 7 bereitgestellt sind.

Die beiden vorzugsweise einteiligen sowie parallel beabstandeten Seitenplatten oder Seitenteile 8 können bevorzugt in zwei höhenbeabstandete Plattenabschnitte 8a , 8b untergliederbar sein, d.h. in einen unteren Abschnitt 8a mit geringerer Tiefe und einen oberen Abschnitt 8b mit größerer Tiefe, mit einem Überhang 8c zwischen dem unteren und oberen Abschnitt. Im Bereich des unteren Abschnitts 8a können die beiden Seitenplatten 8 über eine Frontplatte 11 fest verbunden sein, beispielsweise die beiden Seitenplatten 8 in Querrichtung aussteifend verschweißt oder verschraubt sein.

Ebenso können die Haube 9 und vorzugsweise auch der Gerätesockel bzw. die Bodenplatte 6 an den Ober- und Unterkanten der beiden Seitenplatten 8 festgelegt, beispielsweise über zumindest Teile der Kantenlänge hinweg versteifend angeschweißt sein. Auf diese Weise kann durch die beiden Seitenplatten 8 , die Haube 9 , die Bodenplatte 6 und die Frontplatte 11 ein verwindungssteifes Gehäuse 1 bereitgestellt sein, dessen Inneres an der Rückseite vollständig und an der Vorderseite zumindest im Bereich des oberen Abschnitts 8b zugänglich ist.

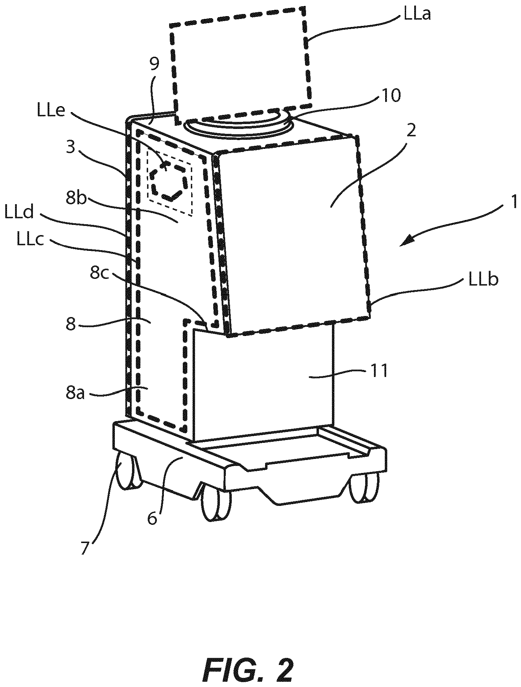

Fig. 2 zeigt das Gerätegehäuse gemäß Fig. 1 mit einem Lichtleiter als lichtgebender Einrichtung gemäß einem ersten bevorzugten Ausführungsbeispiel der Dialysemaschine mit lichtgebender Einrichtung.

In dem in Fig. 2 gezeigten ersten Ausführungsbeispiel wird als lichtgebende Einrichtung wenigstens ein Licht(wellen)leiter bzw. Lichtleiter vorbestimmter Länge zur definierten Beleuchtung innerhalb und/oder außerhalb der Dialysemaschine eingesetzt.

Der Lichtleiter ist ein optisch transparentes und vorzugsweise in Längsrichtung flexibles Element, wie beispielsweise eine (Glas)Faser oder ein Bündel solcher Fasern, eine Röhre oder ein Stab, das bzw. der zum Transport von Licht ausgelegt ist, und kann aus Glas, oder insbesondere für Beleuchtungszwecke vorzugsweise teilweise oder ganz aus zum Beispiel einem flexibel verlegbaren, polymeren Material wie etwa Polymethylmethacrylat (PMMA) oder Polycarbonat (PC) bestehen.

In einer ersten Ausführungsform des Lichtleiters kann dieser so konfiguriert sein, dass die Lichtleitung entweder durch Totalreflexion an einer Grenzfläche seines Umfangs aufgrund eines geringeren Brechungsindex des den Lichtleiter umgebenden Mediums oder durch Verspiegelung der Grenzfläche erreicht wird. In dieser ersten Ausführungsform ist an einem ersten Ende des Lichtleiters stirnseitig Licht über ein Leuchtmittel, beispielsweise zumindest eine Leuchtdiode, vorzugsweise zumindest eine helle bzw. superhelle LED, oder andere geeignete Lichtquellen Licht einkoppelbar, wird das eingekoppelte Licht verlustarm durch den Lichtleiter transportiert, und tritt an einem zweiten Ende des Lichtleiters wieder aus.

In einer zweiten Ausführungsform des Lichtleiters kann dieser mittels oberflächlichen Veränderungen, beispielsweise einer definierten Veränderung an vorbestimmter Stelle oder auch entlang der gesamten Länge, derart konfiguriert (aktiviert) sein, dass an veränderten Stellen an dem ersten Ende eingekoppeltes Licht über die Mantelfläche wieder abgegeben wird. In anderen Worten wird in dieser zweiten Ausführungsform Licht durch partielle Unterbrechung der Totalreflexion quer zur Transportrichtung des Lichts ausgekoppelt, und emittiert daher bei aktiver Lichtquelle der Lichtleiter Licht auch entlang seines Längsverlaufs, wobei entlang des Verlegewegs eines von einer Lichtquelle gespeisten flexiblen Lichtleiters eine oder mehrere Beleuchtungsstellen abgreifbar sein können. Der Lichtaustrag kann dabei allseitig radial bzw. rundum oder auf einer zu einer Veränderungsstelle diametral gegenüberliegenden Seite des Lichtleiters durch die Deckschicht erfolgen. Eine Veränderungsstelle, beispielsweise eine vorbestimmte Schädigung, kann beispielsweise durch gezieltes Aufrauen eines vorbestimmten Umfangsbereichs des Lichtleiters durch etwa mechanisch abtragende Bearbeitung erzeugt werden.

In dem ersten Ausführungsbeispiel ist es mit den vorstehenden ersten und zweiten Ausführungsformen des Lichtleiters möglich, eine gerätezustands- bzw. statusabhängige Beleuchtung, Ausleuchtung und/oder Signalisierung sowohl der Dialysemaschine als auch deren Umgebung bereitzustellen.

Zum einen kann beispielsweise ein Lichtleiter LLa, LLb, LLc, LLd entsprechend einer umlaufenden Gehäusekontur angeordnet sein. Der Lichtleiter LLa, LLb, LLc, LLd ist flexibel verlegbar, d.h. er kann ausgehend von einer Lichteinkopplungsposition an geeigneter Stelle im Inneren des Gehäuses 1 zu einer Gehäusewandung geführt und dort beispielsweise in umlaufende Ausnehmungen (Nuten) an Gehäuseteilen eingelegt bzw. versenkt (LLa, LLb, LLc, LLd), mittels Halteelementen aufflächig kontur- oder randförmig angeordnet (LLa, LLb, LLc, LLd), mit wahlfreier Lichtleitergrafik innenseitig hinter transparenten Wandungsabschnitten verlaufend (LLe), und/oder, gegebenenfalls in Verlängerung, als Freiform ausgebildet gegenüber einer Gehäusewandung vorstehend vorgesehen sein (nicht dargestellt). Die vorstehenden Anordnungsbeispiele sind darüber hinaus miteinander kombinierbar. Eine besondere Beschränkung besteht in dieser Hinsicht nicht, solange beispielsweise minimale Biegeradien des Lichtleiters eingehalten werden können.

Somit ist es beispielsweise möglich, wie in Fig. 2 schematisch angedeutet, mittels eines Lichtleiters LLa entsprechend einer umlaufenden Gehäusekontur einen Rahmen eines sich oberseitig befindenden Monitors oder Bedienfelds der Dialysemaschine an dessen Umfang und/oder stirnseitig auszuleuchten, und/oder einen Lichtleiter entlang des Umfangs des Anschlusssockels 10 zu führen, und/oder einen Lichtleiter LLb entlang der Umfangskontur der Tür/Klappe 2 zu führen, und/oder eine Lichtleitergrafik LLc in zumindest eine der Gehäusewandungen, beispielsweise in eine Seitenwand 8, einzulassen, und/oder eine Lichtleitergrafik LLe hinter einem transparenten bzw. opaken Abschnitt zumindest einer der Gehäusewandungen durchleuchtend zu führen, und oder einen Lichtleiter LLc entlang nur bestimmter Gehäusekonturen sichtbar bzw. Licht auskoppelnd und im Übrigen verborgen bzw. lichtauskopplungslos zu führen.

Der flexible Lichtleiter LLa, LLb, LLc, LLd, LLe kann darüber hinaus mit einkoppelnden Lichtquellen wie beispielsweise LEDs in unterschiedlichen Farben und Intensitäten zusammenwirken. Dadurch sind sanfte Ambientebeleuchtungen ebenso wie Aus- und Beleuchtungen mit starker Leuchtkraft realisierbar. Anwendungsbeispiele an einer Dialysemaschine können beispielsweise eine Notbeleuchtung, ein Nachtlicht, eine Griffbeleuchtung, eine Anschlussbeleuchtung, eine Kanten- und Konturenbeleuchtung, eine Arbeitsplatzbeleuchtung, ein Servicelicht, Gefahrenhinweise, ein Signallicht, eine Objektbeleuchtung, beispielsweise auch betriebsablaufabhängig mitwandernd und/oder in Farbe und/oder Intensität schwankend, und dergleichen sein. Auf vergleichbare Weise ist es möglich, eine partielle Ausleuchtung mit seitlich umlaufendem Lichtleiter zu erzielen.

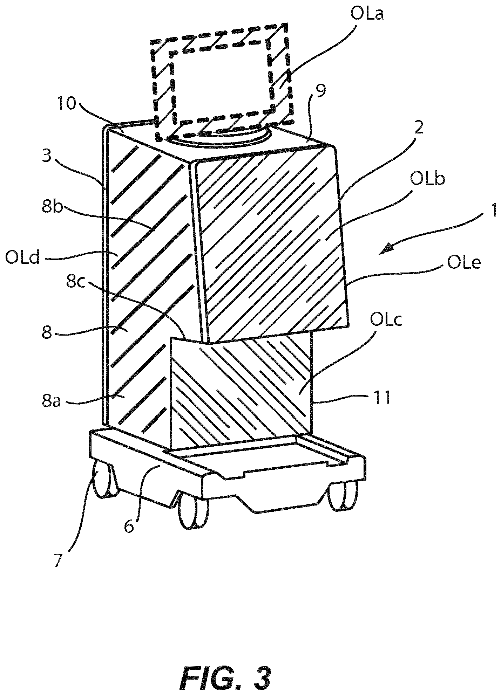

Fig. 3 zeigt das Gerätegehäuse gemäß Fig. 1 mit einer OLED-Einrichtung OLa, OLb, OLc, Old, OLe als lichtgebender Einrichtung gemäß einem zweiten bevorzugten Ausführungsbeispiel der Dialysemaschine mit lichtgebender Einrichtung.

OLED ist an sich bekannt als "organische Leuchtdiode" und kann als solche als eine bestimmte Art einer LED betrachtet werden, die keine Hintergrundbeleuchtung benötigt, durch eine Elektrolumineszenz-Schicht funktioniert und aus Verbundfolie besteht, die wie ein Halbleiter wirkt, wobei der Film zwischen zwei unterschiedlichen Elektroden eingesetzt ist. Die erste Elektrode erscheint dabei als transparent.

Ebenso wie in dem ersten Ausführungsbeispiel ist es in dem zweiten Ausführungsbeispiel möglich, mittels der OLED-Einrichtung OLa, OLb, OLc, Old, OLe alle der in Bezug auf den Lichtleiter LLa, LLb, LLc, LLd, LLe gemäß dem ersten Ausführungsbeispiel beschriebenen Lichtführungs- und/oder Beleuchtungsvariationen zu konfigurieren und zu erzielen. In Fig. 3 ist beispielhaft schematisch angedeutet eine flächige OLED-Einrichtung an einer Monitor(teil)fläche (OLa) und/oder Gehäuse(teil)fläche (OLb, OLc, Old, OLe) vorgesehen. Bei entsprechender Dimensionierung der OLED-Einrichtung OLa, OLb, OLc, Old, OLe kann eine Gehäuseoberfläche vollständig ausgeleuchtet werden.

Als flächiger Lichtleiter bzw. OLED-Einrichtung OLa, OLb, OLc, Old, OLe der vorgenannten Art kann in diesem Fall bevorzugt beispielsweise ein gegebenenfalls mehrteiliges und dann einer Flächengrafik entsprechend geformtes oder zusammengesetztes AMOLED- oder OLED-Flächenmodul als flache, homogen leuchtende Fläche genutzt werden, die mit einer Bauhöhe im Millimeterbereich bis hin zu weniger als 1 mm beispielsweise auf der Gehäuseoberfläche aufliegend festgelegt oder in diese eingelassen sein kann. Aufgrund der selbstleuchtenden, biegsamen und flexiblen Eigenschaften der OLED-Einrichtung OLa, OLb, OLc, Old, OLe sind in diesem Fall, da die gesamte OLED-Einrichtung leuchten kann, auch 360°-Statusanzeigen konfigurierbar.

Es wird angemerkt, dass bei ein und derselben Dialysemaschine ein flexibler Lichtleiter gemäß dem vorgenannten ersten Ausführungsbeispiel und eine OLED-Einrichtung gemäß dem vorgenannten zweiten Ausführungsbeispiel mehrfach und in Kombination, gegebenenfalls mit jeweils unterschiedlicher Farbgebung und/oder Lichtintensität angeordnet und jeweils gemeinsam oder auch separat ansteuerbar sein können. Ferner kann eine manuell, therapie- oder betriebszustandabhängig und/oder sensorgesteuert selbsttätige, beispielsweise umgebungslichtabhängige, Dimmbarkeit, d.h. einstellbare Lichtintensität, einzelner oder aller lichtgebender Einrichtungen vorgesehen sein.

Vorstehend wurde somit eine Dialysemaschine beschrieben, die zumindest ein Bestandteil der Dialysemaschine bildendes Gehäuse 1 zur Aufnahme betrieblicher Komponenten der Dialysemaschine und eine streuscheibenlos lichtgebende Einrichtung LLa bis LLe, OLa bis OLe zur Darstellung eines Betriebs- und/oder Therapiezustands der Dialysemaschine nach dem Umlichtprinzip beinhaltet, wobei die lichtgebende Einrichtung LLa bis LLe, OLa bis OLe in vorbestimmtem Verlauf und/oder mit vorbestimmter Formgebung an zumindest einem Abschnitt des Gehäuses 1 angeordnet ist. Die lichtgebende Einrichtung LLa bis LLe, OLa bis OLe ist ein flexibler Lichtleiter LLa bis LLe, der zumindest eine stirnseitige Lichteinkoppelfläche an einem ersten Ende und zumindest eine stirnseitige Lichtauskoppelfläche an einem zweiten Ende aufweist, oder alternativ eine selbstleuchtende, flächige und/oder flexible OLED-Einrichtung LLa bis LLe, OLa bis OLe.

Die lichtgebende Einrichtung LLa bis LLe, OLa bis OLe ermöglicht eine räumliche Trennung von Lichterzeugungsposition und Lichtausgabeposition und weist gute Energieeffizienz bei gleichzeitig hohen Freiheitsgraden der Konfiguration und geringen Kosten auf. Aufgrund der Eigenschaften und des geringen Raumbedarfs ist die lichtgebende Einrichtung LLa bis LLe, OLa bis OLe selbst bei beengten Raumverhältnissen und an schwierig erreichbare Stellen einfach verlegbar. Durch die vorgenannte räumliche Trennung kann zugunsten besserer Wartbarkeit die Lichterzeugung an einer günstig zu erreichenden Stelle vorgesehen werden. Die in Farbgebung und/oder Intensität variierbare Umlichtcharakteristik verbessert die Wahrnehmung der zu übermittelnden Information durch den Anwender.

Es versteht sich, dass die Erfindung nicht auf die beschriebenen Ausführungsbeispiele beschränkt ist, sondern dass sich innerhalb des durch die nachstehenden Ansprüche definierten Schutzumfangs für den Fachmann gleichwohl naheliegend Kombinationen von zumindest Teilen dieser Ausführungsbeispiele, Modifikationen und Äquivalente ergeben können.

## Patentansprüche

1. Maschine zur extrakorporalen Blutbehandlung, mit
zumindest einem einen Bestandteil der Maschine zur extrakorporalen Blutbehandlung bildenden Gehäuse (1) zur Aufnahme betrieblicher Komponenten der Maschine zur extrakorporalen Blutbehandlung; und
einer streuscheibenlos lichtgebenden Einrichtung (LLa bis LLe; OLa bis OLe), wobei
die lichtgebende Einrichtung (LLa bis LLe; OLa bis OLe) in vorbestimmtem Verlauf und/oder mit vorbestimmter Formgebung an zumindest einem Abschnitt des Gehäuses (1) angeordnet ist;
die lichtgebende Einrichtung (LLa bis LLe; OLa bis OLe) ein flexibler Lichtleiter (LLa bis LLe), der zumindest eine stirnseitige Lichteinkoppelfläche an einem ersten Ende und zumindest eine stirnseitige Lichtauskoppelfläche an einem zweiten Ende aufweist, oder eine selbstleuchtende, flächige und/oder flexible OLED-Einrichtung (OLa bis Ole) ist, **dadurch gekennzeichnet, dass**
zumindest ein Teil der Maschinenoberfläche aufleuchtet; und
die lichtgebende Einrichtung (LLa bis LLe; OLa bis OLe) dazu angeordnet ist, nach dem Prinzip eines Ambient Lights einen Betriebs- und/oder Therapiezustand der Maschine zur extrakorporalen Blutbehandlung auf eine beliebige Oberfläche in der Umgebung der Maschine zur extrakorporalen Blutbehandlung derart zu projizieren, dass der Betriebs- und/oder Therapiezustand der Maschine zur extrakorporalen Blutbehandlung an einer durch die lichtgebende Einrichtung erhellten Fläche im Raum erkennbar ist.

2. Maschine zur extrakorporalen Blutbehandlung nach Anspruch 1, bei der der flexible Lichtleiter (LLa bis LLe) zumindest einen in radialer Richtung Licht auskoppelnden Abschnitt entlang seines Längsverlaufs aufweist.

3. Maschine zur extrakorporalen Blutbehandlung nach einem der vorangehenden Ansprüche, bei der die lichtgebende Einrichtung (LLa bis LLe; OLa bis OLe) dazu angeordnet ist, in einem gesamten Lichtabgabebereich zu leuchten und eine 360°-Statusanzeige zu bilden.

4. Maschine zur extrakorporalen Blutbehandlung nach einem der vorangehenden Ansprüche, bei der die lichtgebende Einrichtung (LLa bis LLe; OLa bis OLe) mit zumindest einem Teil ihrer Ausdehnung entlang einer umlaufenden Gehäusekontur des zumindest einen Gehäuses (1), an zumindest einer Teilfläche des zumindest eines Gehäuses (1), und/oder gegenüber dem zumindest einen Gehäuse (1) vorstehend angeordnet ist.

5. Maschine zur extrakorporalen Blutbehandlung nach einem der vorangehenden Ansprüche, bei der die lichtgebende Einrichtung (LLa bis LLe; OLa bis OLe) mit zumindest einem Teil ihrer Ausdehnung innenseitig der Maschine zur extrakorporalen Blutbehandlung entlang eines transparenten oder opaken Abschnitts einer Gehäusewandung des zumindest einen Gehäuses (1) geführt ist und dazu angeordnet ist, ausgekoppeltes Licht durch den transparenten oder opaken Abschnitt nach außerhalb der Maschine zur extrakorporalen Blutbehandlung abzustrahlen.

6. Maschine zur extrakorporalen Blutbehandlung nach einem der vorangehenden Ansprüche, bei der die lichtgebende Einrichtung (LLa bis LLe; OLa bis OLe) zumindest einen Teil einer Gehäuseoberfläche des zumindest einen Gehäuses (1) bedeckt oder in zumindest einen Teil der Gehäuseoberfläche eingelassen ist.

7. Maschine zur extrakorporalen Blutbehandlung nach Anspruch 6, bei der das zumindest eine Gehäuse (1) ein Gehäuse eines mit einer Rumpfkonfiguration der Maschine zur extrakorporalen Blutbehandlung verbundenen Monitors und/oder Bedienfelds ist.

8. Maschine zur extrakorporalen Blutbehandlung nach einem der vorangehenden Ansprüche, bei der die lichtgebende Einrichtung (LLa bis LLe; OLa bis OLe) eine vorbestimmte Verlaufs-, Linien- und/oder Flächengrafik aufweist.

9. Maschine zur extrakorporalen Blutbehandlung nach einem der vorangehenden Ansprüche, bei der die lichtgebende Einrichtung (LLa bis LLe; OLa bis OLe) dazu angeordnet ist, zumindest abschnittsweise mehrfarbig oder vollflächig einfarbig zu leuchten.

## Claims

1. A machine for extracorporeal blood treatment, comprising
at least one housing (1) for accommodating operational components of the machine for extracorporeal blood treatment which forms part of the machine for extracorporeal blood treatment; and
a light-emitting unit having no diffusion disk (LLa to LLe; OLa to OLe), wherein
the light-emitting unit (LLa to LLe; OLa to OLe) is arranged in a predetermined route and/or with predetermined shaping on at least one portion of the housing (1),
the light-emitting unit (LLa toLLe; OLa to OLe) is a flexible light conductor (LLa to LLe) including at least one end-face light coupling surface at a first end and at least one end-face light output surface at a second end, or is a self-luminous flat and/or flexible OLED unit (OLa to OLe), **characterized in that**
at least a part of the machine surface lights up; and
the light-emitting unit (LLa to LLe; OLa to OLe) is arranged to project, according to the ambient light principle, an operating condition and/or a therapy condition of the machine for extracorporeal blood treatment onto a surface in the environment of said machine in such a way that the operating condition and/or therapy condition of the machine for extracorporeal blood treatment is visible on a surface in the room illuminated by the light-emitting device.

2. The machine for extracorporeal blood treatment according to claim 1, wherein the flexible light conductor (LLa to LLe) includes at least one portion coupling out light in the radial direction along its longitudinal extension.

3. The machine for extracorporeal blood treatment according to any one of the preceding claims, wherein the light-emitting unit (LLa to LLe; OLa to OLe) is arranged so as to emit light in an entire light emission area and to form a 360° status display.

4. The machine for extracorporeal blood treatment according to one of the preceding claims, wherein the light-emitting unit (LLa to LLe; OLa to OLe) is arranged with at least part of its extension along a circumferential housing contour of the at least one housing (1,) on at least one partial surface of the at least one housing (1) and/or projecting from the at least one housing (1).

5. The machine for extracorporeal blood treatment according to one of the preceding claims, wherein the light-emitting unit (LLa to LLe; OLa to OLe) is guided with at least part of its extension on the inside of the machine for extracorporeal blood treatment along a transparent or opaque portion of a housing wall of the at least one housing (1) and is arranged for irradiating output light through the transparent or opaque portion to the outside of the machine for extracorporeal blood treatment.

6. The machine for extracorporeal blood treatment according to one of the preceding claims, wherein the light-emitting unit (LLa to LLe; OLa to OLe) covers at least part of a housing surface of the at least one housing (1) or is embedded in at least part of the housing surface.

7. The machine for extracorporeal blood treatment according to claim 6, wherein the at least one housing (1) is a housing of a monitor and/or an operating field connected to a base configuration of the machine for extracorporeal blood treatment.

8. The machine for extracorporeal blood treatment according to one of the preceding claims, wherein the light-emitting unit (LLa to LLe; OLa to OLe) exhibits a predetermined progression, line and/or area graph.

9. The machine for extracorporeal blood treatment according to one of the preceding claims, wherein the light-emitting unit (LLa to LLe; OLa to OLe) is arranged to emit at least in portions polychromatic or full-surface monochromatic light.

## Revendications

1. Machine pour le traitement sanguin extracorporel, avec au moins un boîtier (1) formant un composant de la machine pour le traitement sanguin extracorporel pour l'accueil de composants opérationnels de la machine pour le traitement sanguin extracorporel ; et
un dispositif lumineux non diffuseur (LLa à LLe ; OLa à OLe), dans laquelle
le dispositif lumineux (LLa à LLe ; OLa à OLe) est disposé selon un tracé prédéterminé et/ou avec une conformation prédéterminée au niveau d'au moins une section du boîtier (1) ;
le dispositif lumineux (LLa à LLe ; OLa à OLe) est un conducteur lumineux flexible (LLa à LLe) qui présente au moins une surface de couplage de lumière frontale au niveau d'une première extrémité et au moins une surface de découplage de lumière frontale au niveau d'une seconde extrémité, ou un dispositif OLED auto-lumineux, plat et/ou flexible (OLa à OLe), **caractérisé en ce que**
au moins une partie de la surface de machine s'illumine ; et
le dispositif lumineux (LLa à LLe ; OLa à OLe) est disposé de façon à projeter selon le principe d'une lumière ambiante un état opérationnel et/ou thérapeutique de la machine pour le traitement sanguin extracorporel sur une surface quelconque dans l'environnement immédiat de la machine pour le traitement sanguin extracorporel de sorte que l'état opérationnel et/ou thérapeutique de la machine pour le traitement sanguin extracorporel est reconnaissable dans l'espace au niveau d'une surface éclairée par le biais du dispositif lumineux.

2. Machine pour le traitement sanguin extracorporel selon la revendication 1, dans laquelle le conducteur lumineux flexible (LLa à LLe) présente au moins une section de découplage de lumière dans la direction radiale le long de son tracé longitudinal.

3. Machine pour le traitement sanguin extracorporel selon l'une quelconque des revendications précédentes, dans laquelle le dispositif lumineux (LLa à LLe ; OLa à OLe) est disposé de façon à éclairer dans une zone d'émission lumineuse globale et à former un affichage de statut à 360°.

4. Machine pour le traitement sanguin extracorporel selon l'une quelconque des revendications précédentes, dans laquelle le dispositif lumineux (LLa à LLe ; OLa à OLe) est disposé avec au moins une partie de son étendue le long d'un contour de boîtier circulaire de l'au moins un boîtier (1), au niveau d'au moins une partie de surface de l'au moins un boîtier (1), et/ou saillant par rapport à l'au moins un boîtier (1).

5. Machine pour le traitement sanguin extracorporel selon l'une quelconque des revendications précédentes, dans laquelle le dispositif lumineux (LLa à LLe ; OLa à OLe) est guidé avec au moins une partie de son étendue côté intérieur de la machine pour le traitement sanguin extracorporel le long d'une section transparente ou opaque d'une paroi de boîtier de l'au moins un boîtier (1) et est disposé pour rayonner une lumière découplée à travers la section transparente ou opaque vers l'extérieur de la machine pour le traitement sanguin extracorporel.

6. Machine pour le traitement sanguin extracorporel selon l'une quelconque des revendications précédentes, dans laquelle le dispositif lumineux (LLa à LLe ; OLa à OLe) recouvre au moins une partie d'une surface de boîtier de l'au moins un boîtier (1) ou est introduit dans au moins une partie de la surface de boîtier.

7. Machine pour le traitement sanguin extracorporel selon la revendication 6, dans laquelle l'au moins un boîtier (1) est un boîtier d'un moniteur et/ou d'un panneau de commande relié à une configuration de coque de la machine pour le traitement sanguin extracorporel.

8. Machine pour le traitement sanguin extracorporel selon l'une quelconque des revendications précédentes, dans laquelle le dispositif lumineux (LLa à LLe ; OLa à OLe) présente un graphique de tracés, de lignes et/ou de surfaces prédéterminé.

9. Machine pour le traitement sanguin extracorporel selon l'une quelconque des revendications précédentes, dans laquelle le dispositif lumineux (LLa à LLe ; OLa à OLe) est disposé de façon à éclairer au moins par zones de manière polychrome ou monochrome sur toute la surface.
